# EUROPEAN PATENT SPECIFICATION

(11) **EP 3 988 016 B1**
(45) Date of publication and mention of the grant of the patent: **17.01.2024**
(21) Application number: 20826621.3
(22) Date of filing: 08.06.2020
(51) Int. Cl.: A61B 5/00, A61B 5/15, A61B 5/155, A61B 5/262

(54) **METHOD OF MANUFACTURING A MICRONEEDLE ARRAY FOR BIOSENSING**
HERSTELLUNGSVERFAHREN FÜR MIKRONADELANORDNUNG FÜR BIOSENSORIK
MÉTHODE DE FABRICATION D'UN BIOCAPTEUR À MICRO-AIGUILLES

(30) Priority: 21.06.2019 KR 20190074059
(43) Date of publication of application: 27.04.2022
(73) Proprietor: Raphas Co., Ltd., Seoul 07793 (KR)
(72) Inventor: LEE, Keun Ho, Goyang-si Gyeonggi-do 10508 (KR); AN, Eun Jin, Seoul 07803 (KR); BAE, Jung Hyun, Seoul 07534 (KR); KIM, Jung Dong, Incheon 22704 (KR); JEONG, Do Hyeon, Seoul 03904 (KR)
(74) Representative: RGTH
(86) International application number: PCT/KR2020/007408
(87) International publication number: WO 2020/256323

(56) References cited:
- WO-A1-2020/060495
- KR-A- 20170 118 669
- KR-A- 20180 006 835
- KR-A- 20190 012 802
- US-A1- 2014 303 471
- CHENJIE, XU: "Conductive microneedles for transdermal delivery in dentistry", Society for biomaterials 2019 annual meeting & exposition, 4 April 2019 (2019-04-04), XP055694595,

## Description

### TECHNICAL FIELD

The present disclosure relates to a method of manufacturing a microneedle array used as such a biosensor.

### BACKGROUND

In a background of the present disclosure, microneedles will be described first. In general, drugs and bioactive substances are orally administered in the form of tablets or capsules. However, various drugs are digested or absorbed in the gastrointestinal tract or lost due to a liver mechanism and accordingly, the drugs and bioactive substances may not be efficiently delivered. In addition, several drugs pass through intestinal mucous membrane and may not be effectively spread. Patient's compliance is also an issue (for example, if a patient needs to take medicine in a regular interval, or critical patents who cannot take medicine).

Other general way of delivering drugs and bioactive substances includes using needles in the related art. It is a more efficient way than oral administration, however, may cause pain on injection sites, a local damage on skin, bleeding, and disease infection on injection sites.

In order to solve matters of oral administration and subcutaneous injection, transdermal administration using patches are used. In the transdermal administration using patches, side effects are small, patient's compliance is high, and drug level in blood remains steady.

Various microstructures including microneedles as one of the transdermal administration described above have been developed. Various metallic materials and various polymer substances are used as the material of the microneedles. In recent years, a biodegradable polymer substance has attracted attention as the material of the microneedles.

A representative method of fabricating a microstructure made of a biodegradable substance is one using a mold. The microstructure, namely the microneedle(s), is formed by initially fabricating a mold having intaglio corresponding to the shape of the microstructure to be formed through the application of a semiconductor manufacturing process, pouring a material of the microstructure into the mold, and separating the material of the microstructure from the mold after the material is coagulated.

For example, in the article "conductive microneedles for transdermal delivery in dentistry" in Society for biomaterials 2019 (XP055694595), a basic molding technique is disclosed in which a molding is fabricated by lithography and casting is applied. Hyaluronic acid (HA) polymer is chosen as a suitable polymer in the design of the microneedle array due to its biocompatible and biodegradable nature. To improve the conductivity of HA microneedle array, a highly water dispersable conductive polymer; poly(3,4-ethylenedioxythiophene)poly(styrenesulfonate) (PEDOT:PSS) is used as a dopant.

The present applicant has a number of domestic and foreign patents related to a microstructure fabricating method using a droplet-born air blowing process, as a new fabricating method that may be substituted for the mold-based microstructure fabricating method. The droplet-born air blowing process will be described in brief below. The droplet-born air blowing process includes: spotting a biocompatible polymer substance having viscosity on a bottom layer of a patch placed on one substrate or the bottom layer of the patch before being coupled to the patch; spotting the same biocompatible polymer substance on a bottom layer of a patch placed on another substrate or the bottom layer of the patch before being coupled to the patch (or omitting this operation); inverting the another substrate upside down; approaching the inverted another substrate toward the one substrate so that the spotted biocompatible polymer substance on the inverted another substrate is brought into contact with the spotted biocompatible polymer substance on the one substrate; stretching (elongating) the biocompatible polymer substances having viscosity which has been brought into contact with each other; performing air blowing after the stretching operation to fix the stretched state of the biocompatible polymer substance; cutting a middle portion of the stretched biocompatible polymer substance so that the same microstructure is formed on each of the one substrate and the another substrate.

In the above, the technical meaning of the microstructure (typically, microneedles) as a means for delivering drugs and bioactive substances, the method of fabricating the microstructure, and the like have been described in brief. In the above description, the microneedle has been used for delivery of drugs into the body, the use of the microneedle is not limited thereto. The microneedle may be used to sense an electrochemical signal inside the body. The microneedle is increasingly being used as a biosensor due to its minimally-invasive properties.

A minimally-invasive biosensor using an existing microneedle patch platform is fabricated by various materials. Various fabricating methods have been known. A well-known material of the microneedle biosensor is a metal. A process of fabricating the microneedle biosensor is similar to a semiconductor manufacturing process. More specifically, the microneedle biosensor made of a metallic material is fabricated through processes such as lithography process, which is a chemical etching using light and heat, and a vacuum deposition process. Photoresist, etchant, and the like that are essential for the lithography process are harmful to the human body.

However, the microneedle biosensor made of a metallic material, which is typically fabricated using the semiconductor manufacturing process as described above, has a matter that products from corrosion of metal inside the skin may cause an inflammatory reaction. In addition, a matter may be generated that the metal microneedle is broken inside the skin.

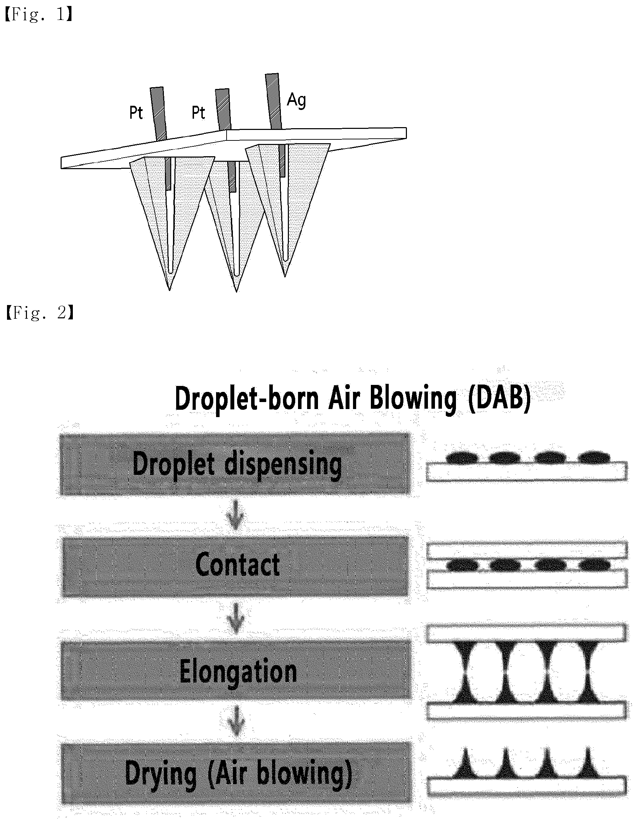

A diagnostic biosensor that may be attached onto the skin is technically convenient to use. Such technical convenience enables a patient to receive a diagnosis remotely without visiting a hospital. Thus, the growth potential of the diagnostic biosensor is very large. In this regard, various attempts have been made to solve the technical matters of the microneedle biosensor made of a metallic material as described above. One of them is a method of inserting a metal electrode into a biocompatible polymer microneedle array as shown in FIG. 1. There is also a method of fabricating a microneedle biosensor with a material obtained by mixing a conductive polymer with a biocompatible polymer. There is also a method of coating a metal film on a microneedle array.

However, the above methods in the related art have some problems. In the method of inserting the metal electrode, an inflammatory reaction may occur due to products caused by corrosion of the metal. In addition, the inserted metal electrode may be broken. In the method of coating the metal film, an inflammatory reaction or the like may occur in the body due to the coating of the metal. In the method of fabricating the microneedle biosensor with the material obtained by mixing the conductive polymer with the biocompatible polymer, a carbon nanotube (CNT) or a reduced graphene oxide (GRO) as a conductive material is mixed with the biocompatible polymer so that the microneedle array has an electrical conductivity. In general, when the biocompatible polymer as a nonconducting substance and the above conductive material are mixed, the electrical conductivity of the conductive material may be lowered, which degrading the function of the biosensor.

The present inventors have continued the development of a new microneedle biosensor, which is capable of providing reliable inspection results by using good minimally-invasive properties of the microneedle and excellent electrically-conductive properties enough to function as the biosensor, and solving several matters caused due to penetration of the metal material into the body, and completed the present disclosure.

### SUMMARY

The present invention provides a method of manufacturing a microneedle array for biosensing as defined in claim 1.

Also disclosed but not claimed are the following aspects.

An aspect provides a new microneedle biosensor which is capable of providing reliable inspection results by using good minimally-invasive properties of the microneedle and excellent electrically-conductive properties enough to function as the biosensor, and solving several matters caused due to penetration of the metal material into the body.

Representative configurations of the present disclosure to achieve the above matters are described below.

According to an aspect, there is provided a manufacturing method for a microneedle array for bio-sensing having a solid structure, and being homogeneous throughout the solid structure. The microneedle array is composed of a material including poly (3,4-ethylendeddioxythiophene ):poly (styrenesulfonate) (PEDOT:PSS).

According to an aspect, the microneedle array for bio-sensing may be composed of the PEDOT:PSS alone. Alternatively, the microneedle array for bio-sensing may be composed of a material including the PEDOT:PSS and biocompatible polymer substances, for example, a hyaluronic acid and a salt thereof.

Further, other additional configurations may be further provided without departing from the technical sprit of the present disclosure.

According to the present disclosure, it is possible to provide a new microneedle biosensor which is capable of providing reliable inspection results by using good minimally-invasive properties of the microneedle and excellent electrically-conductive properties enough to function as the biosensor, and solving several matters caused due to penetration of the metal material into the body.

### BRIEF DESCRIPTION OF THE DRAWINGS

FIG. 1 is a view illustrating a microneedle biosensor in the related art having a structure in which microneedles in which a metal electrode is made of a biocompatible polymer substance are inserted.
FIG. 2 is a view for explaining a droplet-born air blowing, which is a fabricating method used in fabricating a microneedle array according to the present disclosure.
FIG. 3 is a view for explaining a micro-molding process, which is another fabricating method used in fabricating the microneedle array according to the present disclosure, more specifically, a molding method of fabricating a microneedle array by utilizing a replica mold fabricated using a master mold manufactured by the droplet-born air blowing.
FIGS 4 to 6 are photographs obtained by capturing microneedle arrays for biosensor manufactured according to various example embodiments of the present disclosure.

### DETAILED DESCRIPTION

In the following detailed description of the present disclosure, references are made to the accompanying drawings that show, by way of illustration, specific example embodiments in which the present disclosure may be practiced. These example embodiments are described in sufficient detail to enable those skilled in the art to practice the present disclosure. It is to be understood that the various example embodiments of the present disclosure, although different from each other, are not necessarily mutually exclusive. For example, specific shapes, structures and characteristics described herein may be implemented as modified from one example embodiment to another without departing from the present disclosure. Furthermore, it shall be understood that the positions or arrangements of individual elements within each of the example embodiments may also be modified without departing from the present disclosure. Therefore, the following detailed description is not to be taken in a limiting sense. The present invention is defined in the appended claims.

Hereinafter, various preferable example embodiments of the present disclosure will be described in detail with reference to the accompanying drawings to enable those skilled in the art to easily implement the present disclosure.

The present inventors have conducted intensive studies on the development of a material exhibiting electrical conductivity adapted to function as a biosensor, which is capable of preventing any matter in advance from occurring due to the penetration of metal into the human body without using a metallic material, and being fabricated in the form of microneedles without having to use the lithography process in which chemical substances harmful to the human body are used, as briefly described in the Background section of the present disclosure. As a result, the present inventors have fabricated a microneedle biosensor by using a material such as poly (3,4-ethylendeddioxythiophene)poly(styrenesulfonate) (hereinafter referred to as "PEDOT:PSS"). The material PEDOT:PSS is widely used as an electrode material in organic electronics such as an organic photovoltaic device and an organic light emitting diode. Further, the material PEDOT:PSS has a biocompatibility property because it has very little bio-toxicity.

The present inventors have fabricated a microneedle array to be used as a biosensor by using the material PEDOT:PSS in two representative example embodiments as follows. In the first example embodiment, the microneedle array was fabricated using the material PEDOT:PSS alone. In the second example embodiment, the microneedle array was fabricated using a mixture of the material PEDOT:PSS and one or more biocompatible polymer substances. In these two example embodiments, the present inventors have made an attempt to fabricate a microneedle array through two different fabricating methods. The first fabricating method is a droplet-born air blowing process, and the second fabricating method is a micro-molding process. More specifically, the micro-molding process is a process of fabricating a master mold by the droplet-born air blowing process, fabricating a replica mold, and injecting the material PEDOT:PSS alone or the mixture of the material PEDOT:PSS and one or more biocompatible polymer substances into the replica mold:

The droplet-born air blowing process is a process widely known in the related art at the time of filing of the present disclosure. In FIG. 2, there is illustrated a schematic conceptual view of the droplet-born air blowing process. The present applicant has fabricated a microstructure using the droplet-born air blowing process shown in FIG. 2 and commercialized for the first time in the world, which is currently available in the marketplace. The present applicant has a number of patents related to the microstructure. For example, the present applicant has not only domestic patents such as Korean Patent Registration No. 10-1136738 (entitled "method of fabricating a solid microstructure by air-blowing, and the solid microstructure fabricated by the same"), Korean Patent Registration No. 10-125424 (entitled "microstructure fabricating method"), Korean Patent Registration No. 10-1386440 (entitled "solid microstructure fabricated using air-blowing and method of manufacturing the same"), Korean Patent Registration No. 10-1435888 (entitled "method of fabricating biodegradable microneedle using hyaluronic acid"), Korean Patent Registration No. 10-6136069 (entitled "microstructure fabricating method"), and the like, but also a number of foreign patents related to the above domestic patents.

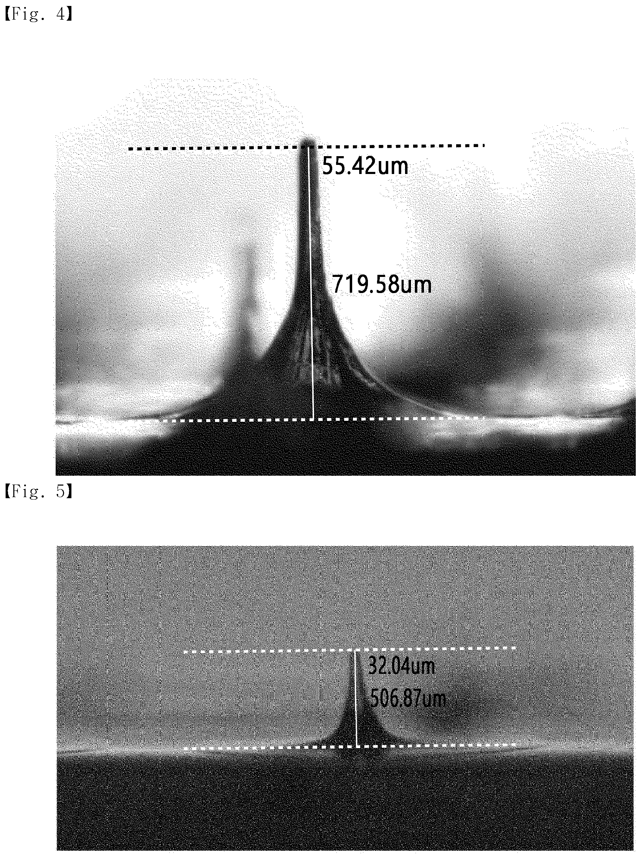

A first operation of the process of fabricating the master mold using the droplet-born air blowing process is spotting a viscous material over a bottom layer placed on a substrate. The bottom layer, which is a material layer as a base for formation of the microstructure, may be obtained by spreading the viscous material widely on the substrate and solidify the same. Alternatively, the bottom layer may be a patch layer that is produced and provided on the substrate by another process. These bottom layers are described in the above-mentioned Korean Patents of the present applicant. Preferably, the viscous material may be a "biocompatible substance". The term "biocompatible substance" used herein refers to a substance that is not toxic to the human body and is chemically inert. Further, preferably, the viscous material may be a substance that is dissolved in a suitable solvent to exhibit viscosity. That is, examples of the substance that exhibits viscosity may include a substance that exhibits viscosity by being melted by heat, or a substance that exhibits viscosity by being dissolved in a solvent. As illustrated in FIG. 4, the viscous material spotted on the substrate as described above is brought into contact with a viscous material spotted on other substrate, or spotted in the same array on the other substrate in the same manner. In this case, as a distance between the two substrates increases, the viscous material in the contact state is stretched to naturally form a shape whose width is narrow in the middle portion and gradually increases toward both end portions, like an hourglass. In this state, air-blowing is performed to solidify the stretched viscous material and subsequently, cut the middle portion having the narrowest width, thus forming a microstructure.

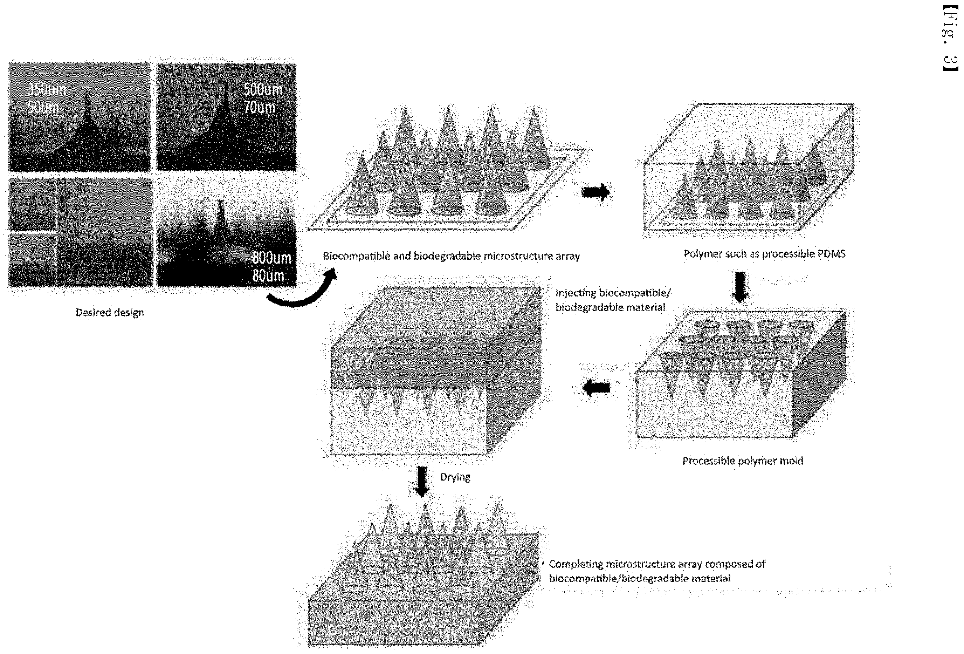

The micro-molding process as the second fabricating method is shown in FIG. 3. Referring to FIG. 3, the process of fabricating the microneedle array by injecting the material PEDOT:PSS alone or the mixture including the material PEDOT:PSS will be described later. In a first operation of the fabricating process illustrated in FIG. 3, the master mold is fabricated. The master mold may be fabricated by the droplet-born air blowing process described with reference to FIG. 2. In a second operation, a polymer substance, such as processible PDMS, is poured into the master mold fabricated by the droplet-born air blowing process. Thereafter, upon polymer substance is solidified, the master mold is removed from the polymer substance. As a result, a replica mold is fabricated. After the fabrication of the replica mold, the material PEDOT:PSS alone or the mixture including the material PEDOT:PSS is injected into the replica mold and then solidified. Subsequently, by removing the solidified material from the replica mold, a microneedle array for biosensor as a final product is obtained. A plurality of replica molds may be fabricated as needed. In this case, by injecting the above material into the plurality of fabricated replica molds and then removing the material from the replica molds at once, several microneedle arrays for biosensor may be formed at once.

### First Example embodiment: Fabricating Microneedle Array with Material PEDOT:PSS alone

Physical properties of an aqueous solution of PEDOT:PSS used to fabricate the microneedle array for biosensor are described in the table below.

### [Table 1]

**[Table 1: Physical properties of a solution of PEDOT:PSS used to fabricate a microneedle array for biosensor]**

| Physical property type of solution PEDOT:PSS | Conditions |
|---|---|
| Viscosity | Viscosity of 2 to 100 mPa*s for |
| | solid content of 1 to 2% |
| Electrical conductivity | 1 to 1,000 S/cm |
| Surface electric resistance | 1 MOhm/sq at maximum |
| Electric resistance | 500 to 5,000 Ohm*cm |
| pH | 1 to 3 |
| Concentration of sodium ion in solution | 10 to 500 ppm |
| Concentration of sulfate in solution | 10 to 100 ppm |
| Ratio of PEDOT to PSS | 1:1 to 10 |

The present inventors have applied the aqueous solution of PEDOT:PSS having the above-described physical properties alone (with no other material mixed in the aqueous solution) to the droplet-born air blowing process and the micro-molding process as described above.

First, it was confirmed that, when the micro-molding process is applied, the microneedle array was formed at a good level. FIG. 4 illustrates photographs of the microneedle array fabricated by the micro-molding process with the aqueous solution of PEDOT:PSS alone. A process of evaporating water in the aqueous solution of PEDOT:PSS was performed in the state in which the aqueous solution of PEDOT:PSS having the physical properties described in the table above is poured in the mold. The microneedle array fabricated in this manner is a solid structure whose interior is completely filled and has homogeneous properties throughout the structure. It was confirmed that the microneedle array illustrated in FIG. 4 meets the length and strength conditions in which the microneedle array may penetrate into the human body at a good depth when attached to the human skin. On the other hand, in order to measure the electrical conductivity of the microneedle, the magnitude of the current flowing through the microneedle was measured while changing the intensity of the voltage to be applied to the microneedle. It was confirmed that the measured magnitude of the current sufficiently satisfies the level suitable for use as the biosensor due to the electrical conductivity of the material PEDOT:PSS itself.

Next, the aqueous solution PEDOT:PSS alone (no other substances are mixed therewith) having the physical properties as indicated in the Table 1 above was applied to the above-mentioned droplet-born air blowing process. The present inventors have found that it is not appropriate to fabricate the microneedle array by the droplet-born air blowing process with merely the aqueous solution of PEDOT:PSS having the above-described physical properties. In the application of the droplet-born air blowing process, a matter was occurred when the aqueous solution of PEDOT:PSS is stretched after the contact. This matter may be caused by the fact that the viscosity of the aqueous solution of PEDOT:PSS is not suitable for application to the droplet-born air blowing process. In addition to the viscosity, there are various physical properties that contribute to form a good microneedle through the droplet-born air blowing process. In the droplet-born air blowing process, rheological properties act collectively. Thus, in order to analyze the cause of failure of the formation of the microneedle array by the droplet-born air blowing process with the aqueous solution PEDOT:PSS having the physical properties as indicated in the Table 1 above, it is considered that a more in-depth study on the rheological properties of the aqueous solution of PEDOT:PSS is necessary.

### Second Example embodiment: Fabricating Microneedle Array with Mixture of One or More Biocompatible Polymer Substances and Material PEDOT:PSS

In consideration of the fact that the formation of the microneedle array fails through the droplet-born air blowing process using merely the aqueous solution of PEDOT:PSS in the first example embodiment, the present inventors repeatedly conducted an experiment to derive a mixing condition in which the formation of the microneedle array is possible through the droplet-born air blowing process in a second example embodiment.

In the second example embodiment, the present inventors set the following materials as biocompatible polymer substances to be mixed with the aqueous solution of PEDOT:PSS: a hyaluronic acid and a salt thereof, polyvinyl pyrrolidone, polyvinyl alcohol, cellulose polymer, dextran, gelatin, glycerin, polyethylene glycol, polysorbate, propylene glycol, povidone, carbomer, gum ghatti, guar gum, glucomannan, glucosamine, dammer resin, rennetcasein, locust bean gum, microfibrillated cellulose, psyllium seed gum, xanthan gum, arabino galactan, arabic gum, alginic acid, gelatin, gellan gum, carrageenan, karaya gum, curdlan, chitosan, chitin, tara gum, tamarind gum, tragacanth gum, furcelleran, pectin or pullulan, lactic acid, polycaprolactone, polylactic acid, poly lactic-co-glycolic acid, hydroxypropyl methylcellulose, hydroxyalkyl cellulose, ethyl hydroxyethyl cellulose, alkyl cellulose, and carboxymethyl cellulose.

The present inventors have found that good microneedle array may be formed by mixing the above-described materials, which are capable of forming a microneedle array by the droplet-born air blowing process, with the aqueous solution of PEDOT:PSS under appropriate mixing conditions. FIG. 5 is a photograph obtained by capturing the microneedle array formed in the above manner. The microneedle array illustrated in FIG. 5 was confirmed to be a solid structure whose interior is completely filled, have homogeneous properties throughout the structure, and meet the length and strength conditions in which the microneedle array may penetrate into the human skin at a good depth when attached to the human skin. Most of water in the aqueous solution of PEDOT:PSS is evaporated in a drying process using air-blowing in the droplet-born air blowing process.

Further, the present inventors conducted the experiment to measure the magnitude of the current flowing through the microneedle while changing the intensity of the voltage to be applied to the microneedle in order to verify the electrical conductivity of the microneedle array having a configuration in which the material PEDOT:PSS formed in the above manner is mixed with different types of biocompatible polymer substances. The magnitude of the current measured for each voltage is shown in the table below.

### [Table 2]

**[Table 2: experimental results obtained by measuring the electrical conductivity of the microneedle array having the configuration in which the material PEDOT:PSS fabricated by the droplet-born air blowing process is mixed with different types of biocompatible polymer substances]**

| | | | |
|---|---|---|---|
| Applied voltage V | 1.5 | 3 | 6 |
| Current (mA) | 0.2 | 0.3 | 0.4 |

In the experimental results indicated in the table above, the microneedle array to be measured is composed of a mixture of PEDOT:PSS and polymeric hyaluronic acid. The above experimental results are evaluated to be a level suitable for use as a biosensor. The present inventors have found that the polymeric hyaluronic acid mixed with the aqueous solution of PEDOT:PSS to which the droplet-born air blowing process is applied satisfies the following conditions.

First, the molecular weight of the hyaluronic acid, which is a biocompatible polymer, and a salt thereof, is 10 to 5,000 kDa, preferably 60 to 130 kDa. The molecular weight of the hyaluronic acid and the salt thereof, which is an object whose electrical conductivity is to be measured in Table 2 above and used to fabricate the microneedle array corresponding to the photograph of FIG. 5, is approximately 80 kDa.

The weight-mixing ratio of the hyaluronic acid and the salt thereof with the aqueous solution of PEDOT:PSS in weight is 1:1 to 100, preferably 1:9 to 49. The weight-mixing ratio of the hyaluronic acid and the salt thereof, which is an object whose electrical conductivity is to be measured in Table 2 above and used to fabricate the microneedle array corresponding to the photograph of FIG. 5, is 1:19.

Such a weight-mixing ratio represents the weight ratio of the hyaluronic acid and the salt thereof with PEDOT:PSS which is in an aqueous solution state. Since PEDOT:PSS is in an aqueous solution state, the above-described weight ratio may be useful information from the viewpoint of producing a mixed solution by mixing the hyaluronic acid and the salt thereof with PEDOT:PSS.

On the other hand, most of the water in the aqueous solution of PEDOT:PSS evaporates away during the micro-molding process, during the droplet-born air blowing process, or during the process of fabricating the microneedle array as a final product. The composition ratio of the microneedle array as a final product in which the water is removed, may be calculated. For example, in a case in which PEDOT:PSS contains 1.5% of solid content, when the hyaluronic acid and the salt thereof are mixed with the aqueous solution of PEDOT:PSS at a weight ratio of 1: 100, the composition ratio of the microneedle array as a final product is 1: 1.5 (HA:PEDOT:PSS).

Meanwhile, the intrinsic viscosity of the hyaluronic acid and the salt thereof is 0.1 to 2 m³/kg.

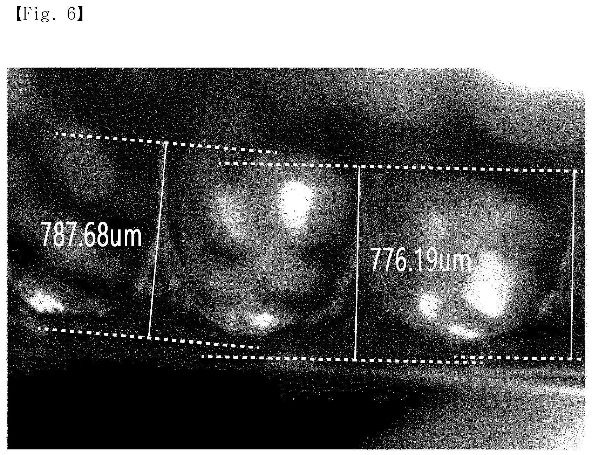

The present inventors made an attempt to form a microneedle array by applying, to the micro-molding process, the same material as that used in the fabricating method using the droplet-born air blowing process. As a result, a microneedle array corresponding to a photograph as illustrated in FIG. 6 was obtained. The microneedle array illustrated in FIG. 6 was confirmed to be a solid structure whose interior is completely filled, have homogeneous properties throughout the structure, and meet the length and strength conditions in which the microneedle array may penetrate into the human skin at a good depth when attached to the human skin. Further, since the same material is used in the droplet-born air blowing process and the micro-molding process, it was confirmed that results obtained by measuring the electrical conductivity are also the same and thus the microneedle array is evaluated to be a level suitable for use as a biosensor.

A microneedle array patch made of a material containing PEDOT:PSS may transmit through the skin in a minimally-invasive manner, and deliver electrical signals generated by undergoing a physicochemical reaction with a specific bio-factor inside the skin, thus functioning as a biosensor. That is, the present inventors have found that the mixture of PEDOT:PSS and the biocompatible polymer substance may function not only as a material of a skin invasion tool (microneedle array patch) but also a material of an electrode capable of transmitting the electrical signals. Further, unlike the product in the related art, the microneedle array patch of the present disclosure has a feature that the material PEDOT:PSS itself or the mixture of PEDOT:PSS and the biocompatible polymer substance is homogeneously spread throughout the structure of the microneedle array, and has the solid structure whose interior is completely filled and in which no artificial clearances occur due to the insertion of the metal electrode or the like. As a result of verification by the present inventors, it was confirmed that such a structural feature according to the present disclosure is very advantageous in securing both stable electrical conductivity that ensures reliable transmission of electrochemical signals inside the body and biochemical stability during which the needle penetrates into the body.

The development of the polymer microneedle biosensor that has biocompatible and electrically-conductive properties and used for minimally-invasive bio-sensing using the mixture containing PEDOT:PSS is advantageous in that complicated and detrimental semiconductor manufacturing processes such as lithography and etching, which are methods of fabricating the existing microneedle-based patch-type biosensor, are not employed. According to the present disclosure, it became possible to produce the microneedle array for biosensor in large quantities by applying the droplet-born air blowing process and the micro-molding process without having to use such complicated and detrimental semiconductor manufacturing processes. As a major advantage of the present disclosure, there may be a case in which a microneedle array for biosensor may be produced more environmentally friendly at a relatively low cost.

The biosensor composed of the microneedle array described in the present specification may penetrate the stratum corneum in a minimally-invasive manner to acquire information about a skin factor inside the skin. By using a microneedle patch platform biosensor, it is possible to continuously monitor transition of accumulation or reduction of disease factors inside the skin in real time. Further, the biosensor may be usefully used to predict or precisely manage development of a specific disease early.

While the present disclosure has been described in the foregoing by way of specific and limited example embodiments and drawings, such as specific components and the like, this is merely provided to aid in a more general understanding of the present disclosure, and the present disclosure is not limited to the foregoing example embodiments, and various modifications and alterations may be made from the substrate to those skilled in the art to which the present disclosure pertains. The invention is defined in the following claims.

## Claims

1. A method of manufacturing a microneedle array for biosensing, the method comprising:
preparing viscous compositions to be applied to a droplet-born air blowing process by mixing a hyaluronic acid and a salt thereof with an aqueous solution of poly (3,4-ethylendeddioxythiophene):poly (styrenesulfonate) (PEDOT:PSS); and
manufacturing the microneedle array by applying the viscous compositions to the droplet-born air blowing process,
wherein the microneedle array has a solid structure and is homogeneous throughout the solid structure, and
in the preparing of the viscous compositions, the hyaluronic acid and the salt thereof are mixed with the aqueous solution of the PEDOT:PSS at a weight ratio of 1:9 to 49, and the hyaluronic acid and the salt thereof have a molecular weight of 60 to 130 kDa.

2. The method of Claim 1, wherein the manufacturing of the microneedle array includes:
placing bottom layers on two different substrates;
spotting the viscous compositions on the bottom layers;
approaching the bottom layers to each other with movement of the two different substrates so that the viscous compositions are brought into contact with each other;
stretching the viscous compositions brought into contact with each other to generate a stretched viscous composition while making a relative distance between the two different substrates far from each other;
performing an air blowing in a state in which the viscous compositions are stretched to fix the stretched viscous composition; and
cutting a middle portion of the stretched viscous composition.

## Patentansprüche

1. Verfahren zum Herstellen einer Mikronadelanordnung zur Biosensorik, wobei das Verfahren umfasst:
Herstellen viskoser Zusammensetzungen, die in einem Luftblasverfahren mit Tröpfchenbildung angewendet werden, durch Mischen einer Hyaluronsäure und eines Salzes davon mit einer wässrigen Lösung von Poly(3,4-ethylendioxythiophen):Poly(styrolsulfonat) (PEDOT:PSS); und Herstellen der Mikronadelanordnung durch Auftragen der viskosen Zusammensetzungen auf das Luftblasverfahren mit Tröpfchenbildung, wobei die Mikronadelanordnung eine feste Struktur aufweist und in der gesamten festen Struktur homogen ist, und bei der Herstellung der viskosen Zusammensetzungen werden die Hyaluronsäure und ihr Salz mit der wässrigen Lösung des PEDOT:PSS in einem Gewichtsverhältnis von 1:9 bis 49 gemischt, und die Hyaluronsäure und ihr Salz weisen ein Molekulargewicht von 60 bis 130 kDa auf.

2. Verfahren gemäß Anspruch 1, wobei das Herstellen der Mikronadelanordnung Folgendes beinhaltet:
Auflegen von Bodenschichten auf zwei verschiedene Substrate;
Aufbringen der viskosen Zusammensetzungen auf die Bodenschichten;
Annäherung der Bodenschichten aneinander durch Bewegung der beiden unterschiedlichen Substrate, so dass die viskosen Zusammensetzungen miteinander in Kontakt gebracht werden;
Dehnen der miteinander in Kontakt gebrachten viskosen Zusammensetzungen, um eine gedehnte viskose Zusammensetzung zu erzeugen, während ein relativer Abstand zwischen den beiden verschiedenen Substraten erzeugt wird;
Durchführen eines Luftblasens in einem Zustand, in dem die viskosen Zusammensetzungen gedehnt sind, um die gedehnte viskose Zusammensetzung zu fixieren; und Schneiden eines mittleren Bereichs der gedehnten viskosen Zusammensetzung.

## Revendications

1. Méthode de fabrication d'un réseau de micro-aiguilles pour la biodétection, comprenant : préparer des compositions visqueuses à appliquer à un processus de soufflage d'air à partir de gouttelettes en mélangeant un acide hyaluronique et un de ses sels avec une solution aqueuse de poly (3,4-éthylendeddioxythiophène):poly (styrenesulfonate) (PEDOT:PSS) ; et fabriquer le réseau de micro-aiguilles en appliquant les compositions visqueuses au processus de soufflage d'air à partir de gouttelettes, dans lequel le réseau de micro-aiguilles a une structure solide et est homogène dans l'ensemble de la structure solide, et lors de la préparation des compositions visqueuses, l'acide hyaluronique et son sel sont mélangés à la solution aqueuse de PEDOT:PSS dans un rapport de poids de 1:9 à 49, et l'acide hyaluronique et son sel ont un poids moléculaire de 60 à 130 kDa.

2. La méthode de la revendication 1, dans laquelle la fabrication du réseau de micro-aiguilles comprend : placer des couches inférieures sur deux substrats différents ; l'application des compositions visqueuses sur les couches inférieures ; rapprocher les couches inférieures l'une de l'autre en déplaçant les deux substrats différents de manière à ce que les compositions visqueuses entrent en contact l'une avec l'autre ; étirer les compositions visqueuses mises en contact l'une avec l'autre pour générer une composition visqueuse étirée tout en éloignant les deux substrats l'un de l'autre ; effectuer un soufflage d'air dans un état dans lequel les compositions visqueuses sont étirées pour fixer la composition visqueuse étirées ; et couper une partie centrale de la composition visqueuse étirée.
